# EUROPEAN PATENT APPLICATION

(11) **EP 1 790 715 A1**
(43) Date of publication of application: **30.05.2007**
(21) Application number: 05782279.3
(22) Date of filing: 08.09.2005
(51) Int. Cl.: C12N 1/20, A23L 1/30, A23L 2/52, A61P 37/08, A61P 43/00, C12R 1/225

(54) **METHOD FOR STABILIZING THE ANTIALLERGIC ACTIVITY OF LACTIC ACID BACTERIA AGANST HIGH-TEMPERATURE TREATMENT, STABILIZED COMPOSITIONS, AND FOOD AND DRINK**

(30) Priority: 10.09.2004 JP 2004264258
(71) Applicant: KIRIN BEER KABUSHIKI KAISHA, Tokyo 104-8288 (JP)
(72) Inventor: KOMEDA, Toshihiro Kirin Beer Kabushiki Kaisha, Yokohama-shi, Kanagawa 236-0004 (JP); IKADO, Kumiko Kirin Beer Kabushiki Kaisha, Yokohama-shi, Kanagawa 236-0004 (JP); YAMAUCHI, Koichiro Kirin Beverage Kabushiki Kaisha, Yokohama-shi, Kanagawa 230-0052 (JP); SAIKI, Akira Kirin Beverage Kabushiki Kaisha, Yokohama-shi, Kanagawa 230-0052 (JP); SADAKARI, Kiyoko Kirin Beverage Kabushiki Kaisha, Yokohama-shi, Kanagawa 230-0052 (JP)
(74) Representative: HOFFMANN EITLE
(86) International application number: PCT/JP2005/016506
(87) International publication number: WO 2006/028164

(57) **Abstract**

The present invention provides a method for stabilizing an antiallergic activity of lactic acid bacteria against a high-temperature treatment and a composition for the stabilization, which can maintain an antiallergic activity of lactic acid bacteria against a high-temperature treatment, and foods and drinks such as drinks having a stabilized antiallergic activity of lactic acid bacteria, and a method for producing the same. The antiallergic activity of lactic acid bacteria can be maintained stably by making the lactic acid bacteria and polyphenols coexit, even when high-temperature treatments are conducted. As polyphenols used in the present invention, polyphenols extracted from a plant such as green tea-derived polyphenols can be used. In the present invention, various lactic acid bacteria having an antiallergic activity can be used, and in particular, lactic acid bacteria having a high antiallergic activity can be advantageously used as such lactic acid bacteria. The method of the present invention can be applied to foods and drinks for which high-temperature heating treatments are conducted in the process of their production, distribution, or when they are taken.

## Description

### Technical Field

The present invention relates to a method for stabilizing an antiallergic activity of lactic acid bacteria against a high-temperature treatment, a composition for the stabilization of an antiallergic activity of lactic acid bacteria against a high-temperature treatment, and foods and drinks such as drinks having a stabilized antiallergic activity of lactic acid bacteria and a method for producing the same.

### Background Art

Allergy is one of the most frequent diseases seen in developed countries. In general, the development mechanism of allergy is classified into four types, type I to type IV, and it is considered that types I, II, and IV are responsible for the development of food allergy. On the other hand, environmental allergies such as hay fever, atopic dermatitis, bronchial asthma, allergic rhinitis, allergic conjunctivitis have the development mechanism of type I.

Type I allergy is characterized by the induction of allergen-specific IgE and the release of chemical mediators such as histamine and leukotriene. For the occurrence of class switching of antibodies in B cells, and the production of IgE antibodies, Th2 cytokines such as IL-4, IL-5, and IL-13 are required. In fact, it has been revealed that Th2 cells are increased in lymphocytes from allergy patients. In brief, allergens which invade from outside the body are presented to T cells, in a state that they are partially bound to MHC class II molecules , by antigen-presenting cells such as dendritic cells and macrophages, and Th2 cells become activated and differentiate. Th2 cytokines released from the Th2 cells induce class switching of B cells, resulting in the production of IgE antibodies, and the IgE antibodies bind to Fc·R on the surface of mast cells in tissues or blood basophils. At the time of the next invasion, allergens are recognized by the IgE antibodies bound to the surface of mast cells or basophils, and cross-linking is formed between the IgE antibodies. With this stimulus as a trigger, the mast cells or the basophils release chemical mediators explosively, and thereby various symptoms of allergy appear.

With regard to the prevention and the treatment of allergy mediated by IgE or chemical mediators, for example, followings are employed: antihistamines that inhibit signaling from the peripheral nerve by binding to histamine receptors in an antagonistic manner with histamines; antiallergic agents for attempting the amelioration of symptoms by diminishing the activity of chemical mediator-producing cells; steroids for ameliorating inflammations by diminishing immunoreactivity; and hyposensitization therapies for inducing tolerance by injecting an allergen itself periodically. However, for all of these, side effects are viewed as problems and there is no crucial effect.

Recently, a method for controlling the production of Th2 cytokines for the purpose of suppressing IgE has been attracting attention. It has been reported that certain bacteria such as tubercle bacillus, hemolytic *streptococcus* and lactic acid bacteria suppress Th2 immunity by enhancing Th1 immunity, resulting in lowered IgE (Clinical Immunology, vol. 32, p. 454, 1999; International Archives of Allergy and Immunology, vol. 115, p. 278, 1998; Japanese Laid-Open Patent Application No. 9-2959).

Among such bacteria, lactic acid bacteria are easy to apply tofoods, etc., from a safety standpoint , and are useful materials. However, there are many kinds of lactic acid bacteria, and not all lactic acid bacteria have a strong effect to enhance Th1 immunity, and therefore, it is indispensable to select useful bacterial strains. As antiallergic lactic acid bacteria, L. rhamnosus LGG strain is publicly known and it is shown that by administration to pregnant women, the development of atopic dermatitis of their children is suppressed (Lancet, vol. 357, p. 1076, 2001). The use of lactic acid bacteria as antiallergic agents is disclosed in several Patent Gazettes as well. For example, Japanese Laid-Open Patent Application No. 9-2959 discloses that mouse lymphocytes are cultured while supplemented with lactic acid bacteria such as *L. acidophilus, L. brevis, L. buchnerii*, and *L. casei,* and the lactic acid bacteria, with which IgE production level during the culture is observed to be 30 ng/ml or less, are used as antiallergic agents. Japanese Laid-Open Patent Application No. 10-309178 discloses the use of bifidobacteria such as *Bifidobacterium infantis, Bifidobacterium breve, Bifidobacterium longum,* and *Bifidobacterium bifidum* as antiallergic agents for treating food allergy in particular. Japanese Laid-Open Patent Application No. 2000-95697 discloses the use of lactic acid bacteria such as *Enterococcus faecalis* and *Lactobacillus reuteri* as inhibitors of type I allergy such as allergic bronchial asthma, allergic rhinitis, and atopic dermatitis.

On the other hand, it is known that the enhancement of Th1 immunity by lactic acid bacteria means the activation of cellular immunity of macrophages, killer T cells and NK cells through the production of IL-12, IFN·gamma, etc., and that it leads to resistance to viral or bacterial infection or the development of cancer. Specifically, IL-12 produced by macrophages contributes to the acquirement of resistance to foreign enemies, cancers, through the differentiation of undifferentiated helper T cells into Th1 cells, and the activation of monocytes, macrophages and NK cells. Therefore, it is suggested that a lactic acid bacterial strain capable of inducing strong IL-12 production can be used as an immunostimulator (Cancer Immunology Immunotherapy, vol. 49, p. 157, 2000; Japanese Laid-Open Patent Application No. 7-228536, Japanese Laid-Open Patent Application No. 2002-80364).

Recently, there are many people who have underwent changes in their body constitution caused by allergy, such as atopic dermatitis and hay fever, and this is recognized as a social problem. Among those people, to patients having particularly severe symptoms , treatments are provided with the use of various pharmaceuticals. However, it is an actual situation that most of the subjects have not received major treatment and that they cope with their symptoms by receiving symptomatic therapies including folk remedy to keep daily life. In view of the situation thus described, studies for suppressing allergies by diet are actively conducted recently. As a result, antiallergic effects are found in components of various foods and drinks such as Japanese basil oil, fish oil, and particular tea polyphenol, and treatments with the use of these components are provided.

As for components of foods and drinks having an antiallergic effect, it has become known that there is the same effect in particular lactic acid bacteria as mentioned above, and some of yogurts and lactic acid bacteria drinks using such lactic acid bacteria have been put into practical use as foods and drinks having an antiallergic effect. Lactic acid bacteria are easy to apply to foods from a safety standpoint, and have been attracting attention as useful materials. For example, there is a disclosure of antiallergic agents containing various lactic acid bacteria as active components wherein lactic acid bacteria are employed as antiallergic agents for facilitating the treatment and the prevention of allergies by decreasing the amount of IgE antibodies responsible for the development of type I allergy (Japanese Laid-Open Patent Application No. 9-2959; Japanese Laid-Open Patent Application No. 2004-26729). In the treatment or the prevention with the antiallergic foods and drinks thus described, it is possible to take the foods and drinks under safe and mild conditions, instead, it is necessary to take the foods and drinks everyday continuously, and the foods and drinks have to be effective at a dose which can be taken continuously.

Examples of applications of lactic acid bacteria to foods include yogurt, cheese and fermented milk, and application to foods and drinks other than dairy products has been also attempted, however, in any case, application has been limited to foods and drinks that need no high-temperature heating treatment. For example, as an application wherein tea components are used together, the followings are disclosed: hot water in which tea leaves are dipped is allowed to cool, *Lactobacillus plantarum* isolated from green tea is added to the cooled hot water, and the resulting mixture is fermented and used for drinks (Japanese Laid-Open Patent Application No. 11-276072); the lactic acid bacteria cultures which have been cultured in a medium containing tea extracts, etc., are used as foods and drinks (Japanese Laid-Open Patent Application No. 2001-190272); a finely crushed plant resource such as tea leaves is used as a culture medium, lactic acid bacteria such as *Lactobacillus plantarium* are added to the medium and cultured, and the resultant culture is used as a fermented food material (Japanese Laid-Open Patent Application No. 2002-330725); lactic acid bacteria are added to a tea-leaf extract solution from which polyphenol has been removed, and cultured, and the resultant culture is used as tea drinks having a sweet floral fragrance and a high γ-aminobutyric acid content (Japanese Laid-Open Patent Application No. 2003-333990).

However, with regard to most foods and drinks including packed drinks which have been distributed recently, high-temperature heating treatments are conducted in the process of their production, distribution, or when they are taken. Therefore, there is a problem that physical properties of lactic acid bacterial cells are changed at the time of the high-temperature heating treatments, so that the antiallergic effect is affected. Consequently, there has been a conventional problem that to foods and drinks for which high-temperature heating treatments are conducted, the utilization of antiallergic activity of lactic acid bacteria is limited. However, neither the presence of the problem thus described, nor the effect of high-temperature heating treatment on the antiallergic ability of lactic acid bacteria has been discussed, and the antiallergic activity of lactic acid bacteria has not been utilized conventionally to foods and drinks for which high-temperature heating treatments are conducted.

On the other hand, bioactivity of polyphenols contained in plants, foods and drinks has been attracting attention, and many studies and reports have been made. For example, as effects of polyphenols in green tea-extracts, antibacterial effect, antioxidant effect, suppressive effect on the elevation of cholesterol levels, inhibitory effect on α amylase activity, etc., are disclosed (Japanese Laid-Open Patent Application No. 2003-138258; Japanese Laid-Open Patent Application No. 60-156614; Japanese Laid-Open Patent Application No. 3-133928; Japanese Laid-Open Patent Application No. 2004-166671). Laxative effect caused by taking lactic acid bacteria and polyphenol simultaneously (Japanese Laid-Open Patent Application No. 2004-155727), and culture in a tea extract solution (Japanese Laid-Open Patent Application No. 2003-333990) are also disclosed. All of these disclosures have focused on the bioactivity of polyphenols, and none of them have referred to interaction with lactic acid bacteria with regard to antiallergic ability.

Patent Document 1: Japanese Laid-Open Patent Application No. 60-156614
Patent Document 2: Japanese Laid-Open Patent Application No. 7-228536
Patent Document 3: Japanese Laid-Open Patent Application No. 9-2959
Patent Document 4: Japanese Laid-Open Patent Application No. 10-309178
Patent Document 5: Japanese Laid-Open Patent Application No. 11-276072
Patent Document 6: Japanese Laid-Open Patent Application No. 2000-95697
Patent Document 7: Japanese Laid-Open Patent Application No. 2001-190272
Patent Document 8: Japanese Laid-Open Patent Application No. 2002-330725
Patent Document 9: Japanese Laid-Open Patent Application No. 3-133928
Patent Document 10: Japanese Laid-Open Patent Application No. 2002-80364
Patent Document 11: Japanese Laid-Open Patent Application No. 2003-138258
Patent Document 12: Japanese Laid-Open Patent Application No. 2003-333990
Patent Document 13: Japanese Laid-Open Patent Application No. 2004-26729
Patent Document 14: Japanese Laid-Open Patent Application No. 2004-155727
Patent Document 15: Japanese Laid-Open Patent Application No. 2004-166671
Non-Patent Document 1: Clinical Immunology, vol. 32, p. 454, 1999; International Archives of Allergy and Immunology, vol. 115, p. 278, 1998
Non-Patent Document 2: Lancet, vol. 357, p. 1076, 2001
Non-Patent Document 3: Cancer Immunology Immunotherapy, vol. 49, p. 157, 2000

### Disclosure of the Invention

### An Object to be Attained by the Invention

The object of the present invention is to provide a method for stabilizing an antiallergic activity of lactic acid bacteria against a high-temperature treatment and a composition for the stabilization of an antiallergic activity of lactic acid bacteria against a high-temperature treatment, which can be applied to foods and drinks for which high-temperature heating treatments are conducted in the process of their production, distribution, or when they are taken, and also to foods and drinks to be stored at room temperature after the high-temperature treatments, in other words, which can maintain an antiallergic activity of lactic acid bacteria against a high-temperature treatment, and foods and drinks such as drinks having a stabilized antiallergic activity of lactic acid bacteria, and a method for producing the same.

### Means for Attaining the Object

In the process of an intensive study for an antiallergic activity of lactic acid bacteria in foods and drinks for which high-temperature treatments are conducted, the present inventors have found that the antiallergic activity of lactic acid bacteria can be maintained stably by making the lactic acid bacteria and polyphenols coexit, even when high-temperature treatments are conducted, and the present invention has been thus completed.

In other words, in the process of a study wherein lactic acid bacteria having an antiallergic ability were added to green tea and then UHT sterilization was conducted, the present inventors have found that polyphenol, a component of green tea, contributes to the stabilization of the antiallergic activity under UHT sterilization and high-temperature storage, and to the antiallergic activity of foods and drinks under room temperature storage after high-temperature treatments, and the present invention has been made. It is possible to apply the method for stabilizing an antiallergic activity of lactic acid bacteria against a high-temperature treatment according to the present invention to a wide range of foods and drinks, in particular, drinks, etc., for which high-temperature heating treatments are conducted in the process of their production, distribution, or when they are taken. It is possible to provide the method for stabilizing an antiallergic activity of lactic acid bacteria against a high-temperature treatment according to the present invention as a composition for the stabilization of an antiallergic activity of lactic acid bacteria against a high-temperature treatment. With the use of the composition, it is possible to give a stabilized antiallergic activity of lactic acid bacteria to foods and drinks for which high-temperature treatments are conducted. The present invention encompasses such composition for the stabilization of an antiallergic activity of lactic acid bacteria against a high-temperature treatment. As polyphenols used in the present invention, polyphenols extracted from a plant such as polyphenols extracted from green tea can be used preferably. However, when polyphenols are contained in foods and drinks in themselves, as in the case of teas, the present invention can be practiced by using those polyphenols, or by supplementing additional polyphenols to those polyphenols if necessary.

Further, in the present invention, it has been found that: drinks, etc., having a more stabilized antiallergic activity of lactic acid bacteria against a high-temperature treatment can be produced by conducting a high-temperature treatment under a particular pH condition, in other words, in a range of pH 4.6 to 6.6, particularly preferably in a range of pH 5.5 to 6.5, during the production of drinks, etc., having a stabilized antiallergic activity of lactic acid bacteria against a high-temperature treatment with the use of the method for stabilizing according to the present invention, and that: drinks, etc., having a more stabilized antiallergic activity of lactic acid bacteria against a high-temperature treatment can be produced by adjusting drinks, etc., prepared by the high- temperature treatment, in a particular pH range, and then filling them into containers and sealing them, during the production of drinks, etc., having a stabilized antiallergic activity of a lactic acid bacterium against a high-temperature treatment with the use of the method for stabilizing according to the present invention, and thus the present invention has been made.

The method for stabilizing an antiallergic activity of lactic acid bacteria against a high-temperature treatment according to the present invention can be applied to various foods and drinks for which high-temperature treatments are conducted, in particular to tea drinks such as green tea, fermented tea, or semi-fermented tea, and the present invention makes it possible to provide drinks having a stabilized antiallergic activity of lactic acid bacteria against a high-temperature treatment. In addition, as lactic acid bacteria, the present invention can use lactic acid bacteria having a high antiallergic activity. The present invention encompasses a method for stabilizing an antiallergic activity of lactic acid bacteria against a high-temperature treatment, a composition for the stabilization of an antiallergic activity of lactic acid bacteria against a high-temperature treatment, and drinks, etc. , having a stabilized antiallergic activity of lactic acid bacteria against a high-temperature treatment, wherein the above-mentioned lactic acid bacteria having a high antiallergic activity are used. Examples of such lactic acid bacteria having a high antiallergic activity include; *L. paracasei* KW3110 and derived strains thereof, deposited as FERM BP-08634 and FERM BP-08635, respectively, in National Institute of Advanced Industrial Science and Technology, International Patent Organism Depositary (address: Central 6, 1-1, Higashi 1-chome, Tsukuba, Ibaraki 305-8566, Japan; date of deposit: February 20, 2004), which is an International Depositary Authority, according to Budapest Treaty on the deposit of microorganism for patent procedure. The method for stabilizing an antiallergic activity of lactic acid bacteria against a high-temperature treatment according to the present invention exerts its effect also in case heat-killed bacteria of lactic acid bacteria are used for giving an antiallergic activity of lactic acid bacteria.

In other words, the present invention specifically comprises: (1) a method for stabilizing an antiallergic activity of a lactic acid bacterium against a high-temperature treatment, wherein the lactic acid bacterium and polyphenols are made to coexist; (2) the method for stabilizing an antiallergic activity of a lactic acid bacterium against a high-temperature treatment according to (1) mentioned above, wherein the polyphenols are polyphenols extracted from a plant; (3) the method for stabilizing an antiallergic activity of a lactic acid bacterium against a high-temperature treatment according to (2) mentioned above, wherein the polyphenols extracted from a plant are polyphenols extracted from green tea; (4) the method for stabilizing an antiallergic activity of a lactic acid bacterium against a high-temperature treatment according to any one of (1) to (3) mentioned above, wherein the lactic acid bacterium has a high antiallergic activity; (5) the method for stabilizing an antiallergic activity of a lactic acid bacterium against a high-temperature treatment according to (4) mentioned above, wherein the lactic acid bacterium having a high antiallergic activity is *Lactobacillus paracasei* KW3110 strain, *Lactobacillus plantarum* KW4110 strain, *Lactobacillus paracasei* KW3925 strain, *Lactobacillus paracasei* KW3926 strain, or *Streptococcus salivarius* KW3210 strain, or a derived strain of these; and (6) the method for stabilizing an antiallergic activity of a lactic acid bacterium against a high-temperature treatment according to (4) or (5) mentioned above, wherein the lactic acid bacterium having a high antiallergic activity is *L. paracasei* KW3110 (FERM BP-08634) or a derived strain thereof (FERM BP-08635).

The present invention also comprises: (7) a composition for stabilization of an antiallergic activity of a lactic acid bacterium against a high-temperature treatment, wherein the lactic acid bacterium according to any one of (1) to (6) mentioned above and the polyphenols according to any one of (1) to (6) mentioned above are made to coexist; and (8) the composition for stabilization of an antiallergic activity of a lactic acid bacterium against a high-temperature treatment according to (7) mentioned above, wherein the composition for stabilization of an antiallergic activity of a lactic acid bacterium against a high-temperature treatment wherein the lactic acid bacterium and the polyphenols are made to coexist, is for adding to a drink.

The present invention further comprises: (9) a food having a stabilized antiallergic activity of a lactic acid bacterium against a high-temperature treatment, wherein the lactic acid bacterium according to any one of (1) to (6) mentioned above and the polyphenols according to any one of (1) to (6) mentioned above are made to coexist; (10) a drink having a stabilized antiallergic activity of a lactic acid bacterium against a high-temperature treatment, wherein the lactic acid bacterium according to any one of (1) to (6) mentioned above and the polyphenols according to any one of (1) to (6) mentioned above are made to coexist; (11) the drink having a stabilized antiallergic activity of a lactic acid bacterium against a high-temperature treatment according to (10) mentioned above, wherein the drink is nondairy and packed in a sealed container; (12) the drink having a stabilized antiallergic activity of a lactic acid bacterium against a high-temperature treatment according to (10) or (11) mentioned above, wherein the drink is a soft drink containing fruit juice or a tea drink and packed in a sealed container; (13) the drink having a stabilized antiallergic activity of a lactic acid bacterium against a high-temperature treatment according to any one of (10) to (12) mentioned above, wherein the lactic acid bacterium according to any one of (1) to (6) mentioned above and the polyphenols according to any one of (1) to (6) mentioned above are made to coexist in the drink by adding the lactic acid bacterium and the polyphenols, or the lactic acid bacterium, to a tea drink; (14) the drink having a stabilized antiallergic activity of a lactic acid bacterium against a high-temperature treatment according to (13) mentioned above, wherein the tea drink is a green tea drink; (15) the drink having a stabilized antiallergic activity of a lactic acid bacterium against a high-temperature treatment according to (13) mentioned above, wherein the tea drink is fermented tea or semi-fermented tea; and (16) the drink having a stabilized antiallergic activity of a lactic acid bacterium against a high-temperature treatment according to any one of (10) to (15) mentioned above, wherein the lactic acid bacterium to be added is in a range of 10⁹ to 10¹¹ bacteria per 100 g of the drink in the drink packed in a sealed container.

The present invention also comprises: (17) a method for producing a drink having a stabilized antiallergic activity of a lactic acid bacterium against a high-temperature treatment, wherein a high-temperature treatment of the drink is conducted in a range of pH 4.6 to 6.6 during production of the drink having an antiallergic activity of a lactic acid bacterium according to any one of (10) to (16) mentioned above; (18) the method for producing a drink having a stabilized antiallergic activity of a lactic acid bacterium against a high-temperature treatment according to (17) mentioned above, wherein the high-temperature treatment of the drink is conducted in a range of pH 5.5 to 6.5; (19) the method for producing a drink having a stabilized antiallergic activity of a lactic acid bacterium against a high-temperature treatment according to (17) or (18) mentioned above, wherein the high-temperature treatment of the drink is a UHT sterilization treatment; (20) a method for producing a drink having a stabilized antiallergic activity of a lactic acid bacterium against a high-temperature treatment, wherein the drink prepared by the high-temperature treatment is adjusted in a range of pH 5.5 to 6.5, and then filled into a container and sealed during production of the drink having an antiallergic activity of a lactic acid bacterium according to any one of (10) to (16) mentioned above; and (21) the method for producing a drink having a stabilized antiallergic activity of a lactic acid bacterium against a high-temperature treatment according to any one of (17) to (20) mentioned above, wherein the drink is a green tea drink.

### Brief Description of Drawings

[Fig. 1] This is a tabular presentation showing lactic acid bacteria which can be used in the present invention and places to obtain them (No. 1).
[Fig. 2] This is a tabular presentation showing lactic acid bacteria which can be used in the present invention and places to obtain them (No. 2).
[Fig. 3] This is a tabular presentation showing lactic acid bacteria which can be used in the present invention and places to obtain them (No. 3).
[Fig. 4] This is a chart showing the changes in the antiallergic activity caused by UHT sterilization when lactic acid bacteria are mixed with each green tea in Examples of the present invention.
[Fig. 5] This is a chart showing the relative antiallergic activity after one week storage at 5°C and 50°C, with regard to the antiallergic activity of lactic acid bacteria in green tea, in Examples of the present invention.
[Fig. 6] This is a chart showing the measurement results of relative activity, with regard to the remaining antiallergic activity of lactic acid bacteria in heat treatments under various pH conditions in Examples of the present invention.
[Fig. 7] This is a chart showing the evaluation results of the antiallergic ability of lactic acid bacteria in green tea in case green tea after UHT sterilization is adjusted to various pHs, and then filled into containers and sealed in Examples of the present invention.

### Best Mode of Carrying Out the Invention

The present invention comprises a method for stabilizing an antiallergic activity of lactic acid bacteria against a high-temperature treatment by making the lactic acid bacteria and polyphenols coexit, a composition for the stabilization of an antiallergic activity of lactic acid bacteria against a high-temperature treatment us ing the method,andfoodsand drinks having a stabilized antiallergic activity of lactic acidbacteria against a high-temperature treatment. The term "high-temperature treatment" used herein includes, for example, temperature treatments which are conducted, at room temperature orhigher, for foods and drinks in the process of their production, distribution, or when they are taken, such as sterilization operations conducted at 50° C or higher, display of products in hot vendors, and UHT sterilization, and which reduce the antiallergic activity of lactic acid bacteria. In the production of drinks, etc., having a stabilized antiallergic activity of lactic acid bacteria according to the present invention, it is preferred that high-temperature treatments are conducted under the condition of pH 4.6 to 6.6. It is more preferred that high-temperature treatments are conducted in a range of pH 5.5 to 6.5.

As polyphenols used in the present invention, polyphenols extracted from a plant such as polyphenols extracted from green tea are preferably exemplified. When polyphenols are contained in foods and drinks in themselves as in the case of teas, it is possible to make lactic acid bacteria and polyphenols coexist by using the polyphenols, and if necessary, by supplementing additional polyphenols to the polyphenols contained in foods and drinks, or by increasing the concentration of polyphenols by enrichment, etc., it is possible to make a necessary amount of polyphenols coexist.

With regard to the preparation of polyphenols from plant-derived materials, the preparation can be appropriately conducted by hot water extraction from plants, etc. As one of methods for obtaining polyphenols of the present invention, green tea extraction is used. Here, the term "green tea extraction" means that extract solutions which have been extracted from tea leaves by hot water or aqueous organic solvents are concentrated and purified, or that the extract solutions are purified directly. In addition, it is also possible to use commercially available concentrated green tea extract solutions such as "Polyphenon" by Mitsui Norin Co., Ltd., "Teafuran" by ITO EN, Ltd., and "Sunphenon" by Taiyo Kagaku Co. , Ltd. Regarding the preferred concentration of polyphenols when lactic acid bacteria and polyphenols are made to coexist in foods and drinks in the present invention, it is preferable that the concentration of total polyphenols is at least in a range of 50 to 130 mg/100 ml, and it is more preferable that the concentration is in a range of 60 to 130 mg/100 ml.

As to a lactic acid bacterium used in the present invention, there is no particular limitation as long as it has an antiallergic activity, and any lactic acid bacterium such as *Lactobacillus, Streptococcus, Lactococcus, Leuconostoc, Enterococcus* can be used. Specific examples of lactic acid bacteria for the present invention include: *Lactobacillus acidophilus, Lactobacillus brevis, Lactobacillus casei, Lactobacillus delbrueckii, Lactobacillus fermentum, Lactobacillus helveticus, Lactobacillus kefir, Lactobacillus paracasei, Lactobacillus plantarum, Lactobacillus rhamnosus, Lactobacillus salivarius, Streptococcus thermophilus*, *Lactococcus lactis, Lactococcus plantarum, Lactococcus raffinolactis, Leuconostoc lactis,* and *Leuconostoc mesenteroides.*

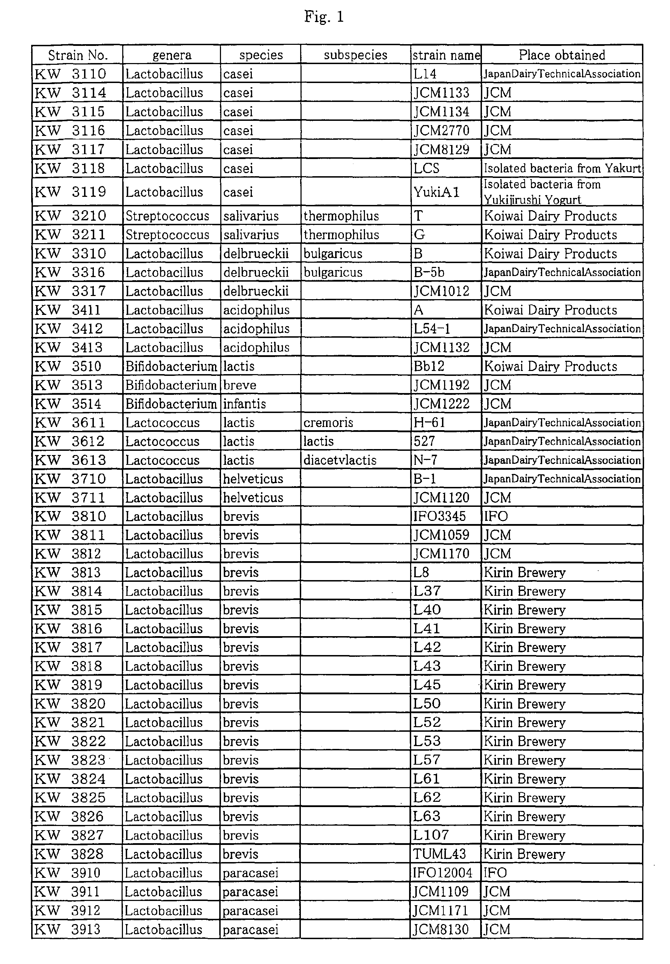

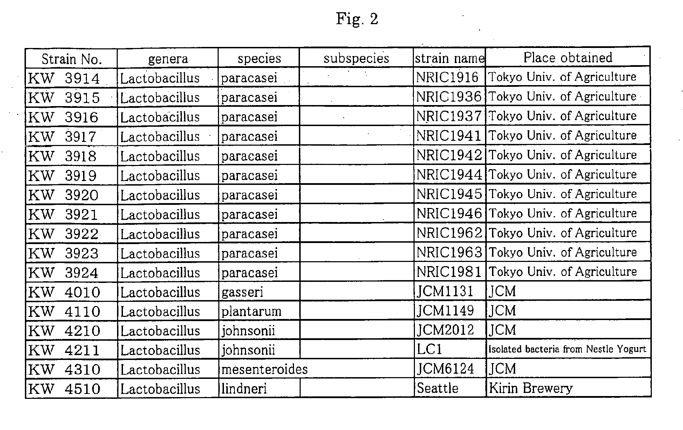

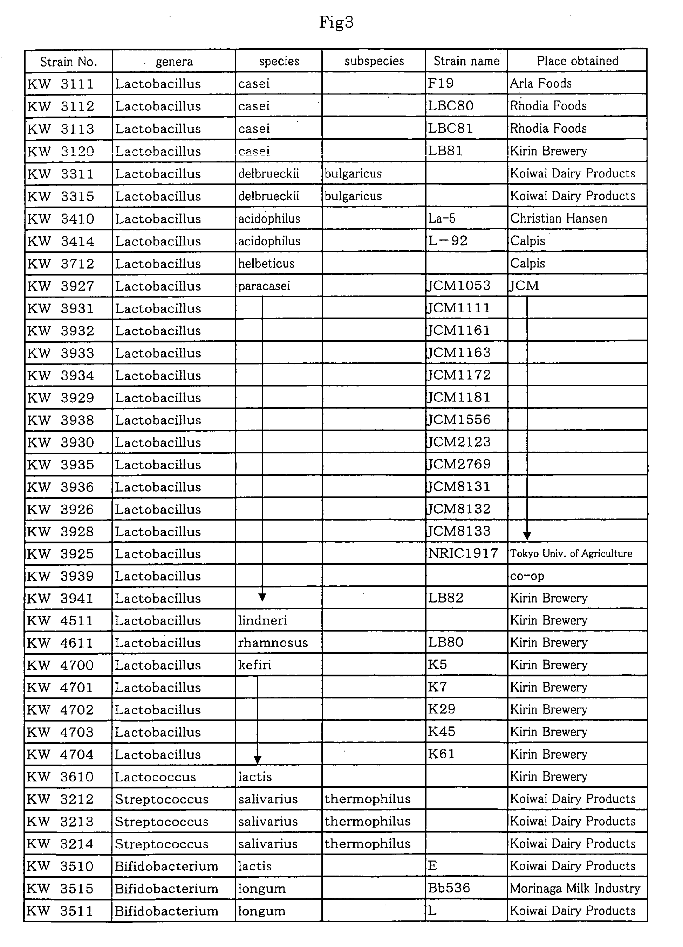

Examples of lactic acid bacteria which can be used in the present invention and places to obtain them are shown in Fig. 1 (No. 1) and Fig. 2 (No. 2). In the present invention, all bacterial strains are identified by code numbers beginning with KW. As these test strains comprise those proprietary isolated, those used in commercially available dairy products, and those obtained from public institutions, etc., the unified identifiers are given to these test strains for the sake of convenience. The relation between each of KW strains and the places to obtain them is as shown in Fig. 1 (No. 1), Fig. 2 (No. 2), and Fig. 3 (No. 3). In "places to obtain" in Figs. 1 and 2, "JCM" refers Japan Collection of Microorganisms, RIKEN BioResource Center, and "IFO" refers former Institution for Fermentation, Osaka (now Incorporated Administrative Agency National Institute of Technology and Evaluation Biological Resource Center (NBRC).

In the present invention, lactic acid bacteria having a high antiallergic activity can be used as lactic acid bacteria having an antiallergic activity. Examples of the lactic acid bacteria having a high antiallergic activity include *Lactobacillus paracasei* KW3110 strain, *Lactobacillus plantarum* KW4110 strain, *Lactobacillus paracasei* KW3925 strain, *Lactobacillus paracasei* KW3926 strain or *Streptococcus salivarius* KW3210 strain, or derived strains of these.

The lactic acid bacteria, for example, *L. paracasei* KW3110 strain can be obtained as *L. casei* L14 strain from Japan Dairy Technical Association. According to the description of Japan Dairy Technical Association, L14 strain is described as *L. casei.* However, when the present inventors analyzed the strain by using RFLP (Restriction Flagment Length Polymorphism) and AFLP (Amplified Flagment Length Polymorphism) with RiboPrinter made by QUALICON, the strain was determined as *L. paracasei,* therefore, the strain is described as *L. paracasei* in the present invention. The *L. paracasei* KW3110 used as an active component of the antiallergic composition in the present invention can be obtained from Japan Dairy Technical Association as mentioned above, and also, the strain has been deposited as FERM BP-08634 in National Institute of Advanced Industrial Science and Technology, International Patent Organism Depositary, which is an International Depositary Authority, according to Budapest Treaty on the deposit of microorganism for patent procedure. Further, a derived strain of *L. paracasei* KW3110 has been deposited as FERM BP-08635 in the International Patent Organism Depositary.

In addition, *L. plantarum* KW4110 strain and *L. paracasei* KW3926 strain, used in the present invention as lactic acid bacteria having a high antiallergic activity, can be obtained from JCM (Japan Collection of Microorganisms, RIKEN BioResource Center) as *L. plantarum* JCM1149 strain and *L. paracasei* JCM8132 strain, respectively, and *L. paracasei* KW3925 strain can be obtained from NRIC (Tokyo University of Agriculture) as *L. paracasei* NRIC1917 strain.

Lactic acid bacteria having an antiallergic activity used in the present invention can be prepared by culturing the lactic acid bacteria in a medium. As the medium used for culture, any medium can be used as long as it is a medium on which the lactic acid bacteria can grow, and animal milk, skim milk, milk whey, MRS medium, GAM medium, BL medium, Briggs Liver Broth and synthetic medium, etc., can be used. Culture temperature can be set from 20°C to 50°C, preferably from 25°C to 42°C. In addition, culture time can be set from 3 hours to 96 hours. After culture, the media can be used as lactic acid bacteria of the present invention as they are, or with an additional treatment if necessary. For example, a preparation of unadulterated bacterial cells prepared by collecting bacteria from a medium after culture by centrifugation, filtering, etc., a spray-dried preparation of the bacterial cells, and a freeze-dried preparation of the bacterial cells can be also used as the lactic acid bacteria of the present invention. In addition to the lactic acid bacterial cells mentioned above, the lactic acid bacteria of the present invention can contain other components. Examples of such other components include additives such as excipients, and components of a medium to be hereinafter described.

A specific example of preparation of lactic acid bacteria having a high antiallergic activity used in the present invention is shown below:

### (Preparation of the lactic acid bacterial cells, KW3110 strain)

For culture of the lactic acid bacteria, a culture medium (1% of glucose, 1% of yeast extract S (Takeda-Kirin Foods Corporation), 50 ppm of MgSO₄·7H₂O, 50 ppm of MnSO₄·5H₂O) was used. Preculture was conducted as follows: a lactic acid bacterium, KW3110 strain, was inoculated to 10 ml of the culture medium, and left for 20 to 24 hours at 37° C. Main culture was conducted as follows: 0.6 ml of preculture solution was inoculated to 120 ml of the culture medium, and cultured with the use of a 200 ml fermentor (model BMJ-25, Able) at 28° C. The ventilation volume was 0.12 L/min, and the stirring speed was 500 rpm. By using 25% NaOH solution, pH was controlled not to fall below pH 4.5. The culture was conducted for 48 hours.

After the culture was finished, bacterial cells were collected by conducting centrifugation at 8500 × g for 10 minutes. The centrifugate was suspended in 30 ml of sterile distilled water, and bacterial cells were collected by conducting centrifugation at 8500 × g for 10 minutes. By repeating this washing operation three times , components derived from the medium were removed. Washed bacterial cells were suspended in 10 ml of sterile water, treated in an autoclave at 100° C for 30 minutes, and then freeze-dried. Finally, 50 to 70 mg of dried lactic acid bacterial cells was obtained. The antiallergic activity of the obtained lactic acid bacterial cells was confirmed.

The method for stabilizing an antiallergic activity of lactic acid bacteria against a high-temperature treatment according to the present invention, wherein the lactic acid bacteria and polyphenols are made to coexist, can be applied to a wide range of foods and drinks for which high-temperature heating treatments are conducted in the process of their production, distribution, or when they are taken. The composition of the present invention can be added to various kinds of foods and drinks as a composition for the stabilization of an antiallergic activity of lactic acid bacteria against a high-temperature treatment, wherein lactic acid bacteria and polyphenols are made to coexist, and can give a stabilized antiallergic activity to foods and drinks.

The method for stabilizing an antiallergic activity of lactic acid bacteria against a high-temperature treatment according to the present invention, wherein the lactic acid bacteria and polyphenols are made to coexist, can be applied advantageously, in particular, when giving a stabilized antiallergic activity to drinks. As such drinks, tea-based drinks or non-tea-based drinks are exemplified. As particularly preferred polyphenols to be blended in that case, polyphenols extracted from a plant such as polyphenols extracted from green tea are exemplified. The polyphenols extracted from green tea are exemplified as particularly preferred polyphenols to be blended with extract solutions of tea selected from green tea, semi-fermented tea, and fermented tea; and non-tea-based drinks as objects of blending. Among these blending combinations, it is preferable to make a drink wherein the green-tea extract mentioned above is blended with an extract solution of tea, and it is particularly preferable to make a green tea drink wherein the green-tea extract mentioned above is blended with an extract solution of green tea.

As the semi-fermented tea and the fermented tea, oolong tea and black tea are exemplified, respectively. Further, as non-tea-based drinks, for example , soft drinks such as carbonated drinks, drinks containing fruit extracts, juice containing vegetable extracts, near water drinks, sports drinks, and diet drinks are exemplified. In case drinks to which a stabilized antiallergic activity has been given are commercialized as drinks packed in sealed containers with the use of the present invention, it is preferable to adjust the number of lactic acid bacteria to be added to the drinks in a range of 10⁹ to 10¹¹ bacteria per 100 g of the drinks.

In the present invention, as drinks of the present invention with which lactic acid bacteria having a high antiallergic activity are blended, various kinds of drinks are exemplified as mentioned above. In the production of those drinks, any of saccharides, flavoring agents, fruit juice, food additives, etc., used for ordinary formulation of drinks can be used. With regard to the production of drinks, it is possible to refer to existing reference books, for example , "Newly-Revised Edition: Soft Drinks" (KORIN PUBLISHING CO., LTD.), etc.

In the present invention, as particularly suitable drinks for blending, drinks which do not containing milk components, in other words, for example, soft drinks containing fruit juice, or nondairy drinks such as tea drinks mentioned above are particularly preferable from the viewpoint of storage and stabilization of the antiallergic effect and the products. The drinks, etc. , having a stabilized antiallergic activity of lactic acid bacteria according to the present invention can be commercialized as "drinks packed in sealed containers" by filling the drinks into containers and sealing them. In that process, it is preferable to adjust the drinks prepared by a high-temperature treatment in a range of pH 5.5 to 6.5, and then fill the drinks into containers.

Hereinafter, the present invention is described more specifically with reference to Examples. The technical scope of the present invention, however, is not limited to these exemplifications.

### Example 1

Extraction was conducted to 100 g of green tea leaves (mainly comprised of kabuse-cha (shade-grown tea) and gyokuro (refined green tea)) for about 6 minutes with 3.5 kg of hot water at 80° C in which 3 g of ascorbic acid had been dissolved. The extract solution was filtered through a mesh to remove tea leaves , and then centrifugated. To the supernatant of the centrifugate, 5 g of sodium hydrogen carbonate and 3 g of ascorbic acid were added, and water was added to make the resultant solution 12 kg in total. This green tea solution was designated as the basic green tea (1). A solution prepared by adding 8 g of polyphenon 70A (Mitsui Norin Co. , Ltd.) to (1) was designated as the basic green tea + polyphenon 70A (2), a solution in which twice as much supernatant of the extract solution after centrifugation as (1) was used was designated as the green tea with double amount of tea-leaf extract solution (3), and a solution in which the supernatant used was prepared by adding 18 g of PVPP to the extract solution before centrifugation in (1), stirring and centrifuging the resultant solution, was designated as the PVPP-treated green tea (4).

The pHs of (1) to (4) were 6.0 to 7.0, and the total polyphenol amounts in (1) to (4) were quantitated by iron tartrate method using ethyl gallate as a standard solution. (Reference: Tea Research Journal 71 (1990), Method of Tea Analysis: Colorimetric Determination of Tannin Level). The quantitation results are shown in Table 1.

**[Table 1]**

| Samples | Total polyphenol amount (mg/100 ml) |
|---|---|
| Basic green tea (1) | 57.5 |
| Basic green tea + polyphenon 70A (2) | 122 |
| Green tea with double amount of tea-leaf extract solution (3) | 111 |
| PVPP-treated green tea (4) | 9.93 |

To the above-mentioned green teas (1) to (4), 0.02% of dried bacterial cells of lactic acid bacterium *Lactobacillus paracasei* KW3110 strain (heat-killed bacterium: obtained from Functional Food Division, Kirin Brewery Co., Ltd.) were added and UHT sterilization was conducted. The sterilization was conducted under the conditions of 135° C for 30 seconds and 137° C for 30 seconds, and each tea was hot-pack filled into a 500 ml PET bottle. From 25 ml of green tea after sterilization, precipitate of lactic acid bacteria was obtained by centrifugation at 8000 rpm for 10 minutes. The precipitate was washed with PBS (-), and then centrifuged again, and the resultant precipitate was suspended in 5 ml of PBS (-).

The antiallergic activity of lactic acid bacteria *in vitro* was determined by measuring the amount of IL-12 released into a medium when mixed culture with mouse spleen lymphocytes was conducted. To BALB/c mice (Charles River Laboratories), 7 to 10 weeks of age, 1 mg of ovalbumin (OVA) was intraperitoneally injected together with 2 mg of aluminum hydroxide, which is an adjuvant, on day 0 and day 6. The mice were dissected on day 13, the spleens were isolated, and lymphocytes were prepared. The spleen lymphocytes were suspended at a cell concentration of 2.5 × 10⁶ cells/ml in RPMI1640 (SIGMA) medium to which FCS (Roche) and OVA were added to the final concentrations of 10% and 1 mg/ml, respectively. The lactic acid bacteria were added to the medium to the concentration of 0.25 µg/ml, and cultured for one week at 37°C and CO₂ concentration of 5%. Culture supernatant was collected by centrifugation, and the amount of IL-12 produced was measured with the use of Opt EIA ELISA Set (Becton Dickinson).

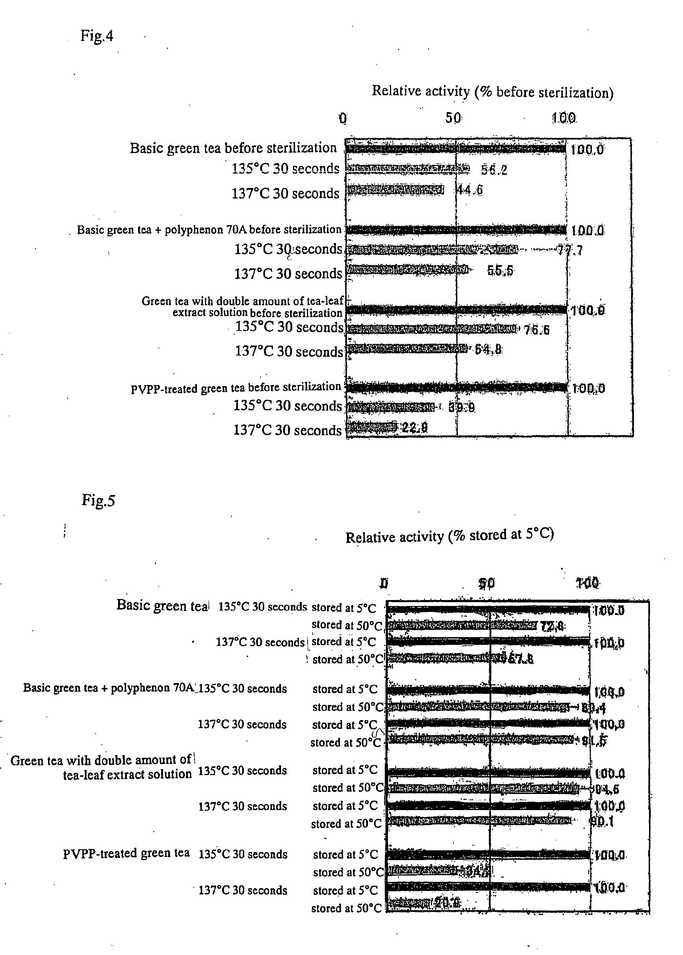

The changes in the antiallergic activity caused by the UHT sterilization when the lactic acid bacteria were mixed with each green tea are shown in Fig. 4. The relative antiallergic activity after the UHT sterilization in comparison to that before the sterilization is shown. In both sterilization conditions, remaining activity after the UHT sterilization of the basic green tea + polyphenon 70A (2) and the green tea with double amount of tea-leaf extract solution was 10 to 20% higher than that of the basic green tea (1) as a result. The remaining antiallergic activity of the PVPP-treated green tea (4) was 10 to 20% lower than that of the basic green tea (1) as a result. It was considered that there was correlation between the levels of the remaining antiallergic activity and the polyphenol contents (tannin contents) shown in Table 1.

### Example 2

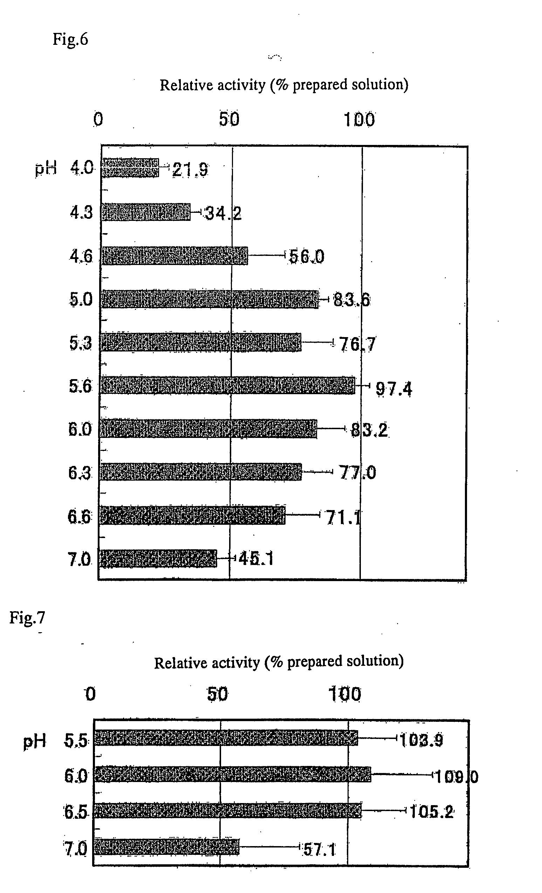

Four kinds of green tea prepared in Example (1) were stored at 5° C and 50° C for one week, and the anitallergic activity of lactic acid bacteria in the green tea after storage was evaluated. The experiment was conducted in a same manner as in Example (1). The relative antiallergic activity after one week storage at 50° C to at 5°C was shown in Fig. 5.

In both sterilization conditions, there was little change in the antiallergic activity between storage temperatures with regard to the basic green tea + polyphenon 70A (2) and the green tea with double amount of tea-leaf extract solution (3), while lowered antiallergic activity was observed in the basic green tea (1), and significantly lowered antiallergic activity was observed in the PVPP-treated green tea (4). The advantage of high polyphenol content was confirmed also in stability in storage at 50° C, as well as the antiallergic activity immediately after the UHT sterilization in Example (1). As the advantage in stability in storage at 50° C was confirmed, it is considered that the prolonged stability in storage at room temperature is high as well.

### Example 3

To 50 mM of citric acid/sodium citrate buffers at pH 4.0, 4.3, 4.6, 5.0, 5.3, 5.6, 6.0, 6.3, 6.6, and 7.0, 0.025% of dried bacterial cells of lactic acid bacterium *Lactobacillus paracasei* KW3110 strain were added, and the resultant solutions were heat-treated at 95°C for 180 minutes. After heating, lactic acid bacterial precipitates were obtained by centrifugation, and the antiallergic ability of the lactic acid bacterium was evaluated. The experiment was conducted in a same manner as in Example (1). The changes in the antiallergic ability caused by the heat treatment when the lactic acid bacterium was mixed with buffers at each pH are shown in Fig. 6. The remaining activity resulted from the heat treatment was pH-dependent. The remaining activity of the buffer at pH 7.0 was about 40% lower than that of the buffer at pH 6.0 as a result. This result was the same as that of Example 4, when green tea was mixed.

### Example 4

Extraction was conducted to 100 g of green tea leaves (mainly comprised of kabuse-cha and twig tea) for about 6 minutes with 3.5 kg of hot water at 80° C in which 3 g of ascorbic acid had been dissolved. The extract solution was filtered through a mesh to remove tea leaves, and centrifugated. To the supernatant of the centrifugate, 3 g each of sodium hydrogen carbonate, ascorbic acid and green tea extract, and 2 g of dried bacterial cells of lactic acid bacterium *Lactobacillus paracasei* KW3110 strain were added, and the resultant solution was made to up to 12 kg with water. This solution was designated as the green tea (1): pH = 6.0. Solutions whose pHs were adjusted to 5.5, 6.5, and 7.0, prepared by changing the amount of sodium hydrogen carbonate added to the green tea (1) mentioned above, were designated as the green teas (2), (3), and (4), respectively. The green teas (1) to (4) were hot-pack filled into 350 ml PET bottles after UHT sterilization. From the green teas after the sterilization, lactic bacterial precipitates were obtained by centrifugation, and the antiallergic ability of the lactic acid bacterium in the green teas was evaluated. The experiment was conducted in a same manner as in Example (1). The changes in the antiallergic ability caused by the UHT sterilization when the lactic acid bacterium was mixed with each green tea are shown in Fig. 7. The remaining activity resulted from the UHT sterilization was comparable among green teas at pH 5.5, 6.0, and 6.5. On the other hand, the remaining activity of the green tea at pH 7.0 was about 50% lower than that of other green teas as a result. It was confirmed that there was correlation between the level of remaining antiallergic ability and pH.

### Industrial Applicability

The present invention provides a method for stabilizing an antiallergic activity of lactic acid bacteria against a high-temperature treatment. By the present invention, the stabilized antiallergic activity of lactic acid bacteria can be given to a wide range of foods and drinks for which high-temperature heating treatments are conducted in the process of their production, distribution, or when they are taken, and to a wide range of foods and drinks to be stored at room temperature after the high-temperature treatments, and the activity can be stabilized and maintained. The present invention can be advantageously applied to drinks such as teas, which contain a lot of polyphenols originally, and the present invention makes it possible to provide drinks having a stabilized antiallergic activity of lactic acid bacteria. As lactic acid bacteria used, lactic acid bacteria having a high antiallergic activity can be used as a preferable aspect in the present invention. With the use of the lactic acid bacteria, foods and drinks with stabilized activity having a high antiallergic function can be provided.

## Claims

1. A method for stabilizing an antiallergic activity of a lactic acid bacterium against a high-temperature treatment, wherein the lactic acid bacterium and polyphenols are made to coexist.

2. The method for stabilizing an antiallergic activity of a lactic acid bacterium against a high-temperature treatment according to claim 1, wherein the polyphenols are polyphenols extracted from a plant.

3. The method for stabilizing an antiallergic activity of a lactic acid bacterium against a high-temperature treatment according to claim 2, wherein the polyphenols extracted from a plant are polyphenols extracted from green tea.

4. The method for stabilizing an antiallergic activity of a lactic acid bacterium against a high-temperature treatment according to any one of claims 1 to 3, wherein the lactic acid bacterium has a high antiallergic activity.

5. The method for stabilizing an antiallergic activity of a lactic acid bacterium against a high-temperature treatment according to claim 4, wherein the lactic acid bacterium having a high antiallergic activity is *Lactobacillus paracasei* KW3110 strain, *Lactobacillus plantarum KW4110strain,Lactobacillus paracasei* KW3925 strain, *Lactobacillus paracasei* KW3926 strain, or *Streptococcus salivarius* KW3210 strain, or a derived strain of these.

6. The method for stabilizing an antiallergic activity of a lactic acid bacterium against a high-temperature treatment according to claim 4 or 5, wherein the lactic acid bacterium having a high antiallergic activity is *L. paracasei* KW3110 (FERM BP-08634) or a derived strain thereof (FERM BP-08635).

7. A composition for stabilization of an antiallergic activity of a lactic acid bacterium against a high-temperature treatment, wherein the lactic acid bacterium according to any one of claims 1 to 6 and the polyphenols according to any one of claims 1 to 6 are made to coexist.

8. The composition for stabilization of an antiallergic activity of a lactic acid bacterium against a high-temperature treatment according to claim 7, wherein the composition for stabilization of an antiallergic activity of a lactic acid bacterium against a high-temperature treatment wherein the lactic acid bacterium and the polyphenols are made to coexist, is for adding to a drink.

9. A food having a stabilized antiallergic activity of a lactic acid bacterium against a high-temperature treatment, wherein the lactic acid bacterium according to any one of claims 1 to 6 and the polyphenols according to any one of claims 1 to 6 are made to coexist.

10. A drink having a stabilized antiallergic activity of a lactic acid bacterium against a high-temperature treatment, wherein the lactic acid bacterium according to any one of claims 1 to 6 and the polyphenols according to any one of claims 1 to 6 are made to coexist.

11. The drink having a stabilized antiallergic activity of a lactic acid bacterium against a high-temperature treatment according to claim 10, wherein the drink is nondairy and packed in a sealed container.

12. The drink having a stabilized antiallergic activity of a lactic acid bacterium against a high-temperature treatment according to claim 10 or 11, wherein the drink is a soft drink containing fruit juice or a tea drink and packed in a sealed container.

13. The drink having a stabilized antiallergic activity of a lactic acid bacterium against a high-temperature treatment according to any one of claims 10 to 12, wherein the lactic acid bacterium according to any one of claims 1 to 6 and the polyphenols according to any one of claims 1 to 6 are made to coexist in the drink by adding the lactic acid bacterium and the polyphenols , or the lactic acid bacterium, to a tea drink.

14. The drink having a stabilized antiallergic activity of a lactic acid bacterium against a high-temperature treatment according to claim 13, wherein the tea drink is a green tea drink.

15. The drink having a stabilized antiallergic activity of a lactic acid bacterium against a high-temperature treatment according to claim 13, wherein the tea drink is fermented tea or semi-fermented tea.

16. The drink having a stabilized antiallergic activity of a lactic acid bacterium against a high-temperature treatment according to any one of claims 10 to 15, wherein the lactic acid bacterium to be added is in a range of 10⁹ to 10¹¹ bacteria per 100 g of the drink in the drink packed in a sealed container.

17. A method for producing a drink having a stabilized antiallergic activity of a lactic acid bacterium against a high-temperature treatment, wherein a high-temperature treatment of the drink is conducted in a range of pH 4.6 to 6.6 during production of the drink having an antiallergic activity of a lactic acid bacterium according to any one of claims 10 to 16.

18. The method for producing a drink having a stabilized antiallergic activity of a lactic acid bacterium against a high-temperature treatment according to claim 17, wherein the high-temperature treatment of the drink is conducted in a range of pH 5.5 to 6.5.

19. The method for producing a drink having a stabilized antiallergic activity of a lactic acid bacterium against a high-temperature treatment according to claim 17 or 18, wherein the high-temperature treatment of the drink is a UHT sterilization treatment.

20. A method for producing a drink having a stabilized antiallergic activity of a lactic acid bacterium against a high-temperature treatment, wherein the drink prepared by the high-temperature treatment is adjusted in a range of pH 5.5 to 6.5, and then filled into a container and sealed during production of the drink having an antiallergic activity of a lactic acid bacterium according to any one of claims 10 to 16.

21. The method for producing a drink having a stabilized antiallergic activity of a lactic acid bacterium against a high-temperature treatment according to any one of claims 17 to 20, wherein the drink is a green tea drink.
